# FASCICULE DE BREVET EUROPEEN

(11) **EP 0 428 676 B1**
(45) Date de publication et mention de la délivrance du brevet: **02.07.1997**
(21) Numéro de dépôt: 90908521.9
(22) Date de dépôt: 29.05.1990
(51) Int. Cl.: A61M 1/16

(54) **GESTION DE PARAMETRES RELATIFS A UN TRAITEMENT DE DIALYSE**
VERARBEITUNG UND SPEICHERUNG VON DIALYSEPARAMETERN
MANAGEMENT OF PARAMETERS RELATING TO A DIALYSIS TREATMENT

(30) Priorité: 31.05.1989 IT 6742189
(43) Date de publication de la demande: 29.05.1991
(73) Titulaire: HOSPAL AG, 4008 Basel (CH)
(72) Inventeur: GHIRALDI, Andrea, I-46025 Poggio Rusco-Mantova (IT)
(74) Mandataire: Lejeune, Daniel
(86) Numéro de dépôt international: EP9000906
(87) Numéro de publication internationale: WO9014850

(56) Documents cités:
- EP-A- 0 251 520
- WO-A-80/02376
- US-A- 4 739 492

## Description

La présente invention concerne un procédé de gestion de paramètres relatifs à un traitement de dialyse et une machine de dialyse apte à mettre en oeuvre un tel procédé.

Le procédé et la machine de dialyse auxquels on se réfère conviennent particulièrement bien, mais non exclusivement, aux traitements de dialyse à domicile, pour lesquels on ne prévoit pas en général d'utiliser une personne spécialisée (médecin ou infirmière) de façon continue pour surveiller le déroulement du traitement.

Il est évident que, au moins du point de vue théorique, le contrôle d'une machine de dialyse peut être effectué à distance et d'une façon continue, à l'aide d'un dispositif récepteur disposé dans un poste de commande centralisé en connectant ce dispositif récepteur avec la machine de dialyse, par exemple à l'aide d'une ligne téléphonique.

Le document EP 0 251 520 décrit un système de contrôle de la santé d'un patient comprenant un ordinateur domestique disposé au domicile du patient. Cet ordinateur domestique est relié par des moyens de transmission de données à une unité centrale.

L'ordinateur domestique est programmé pour demander au patient d'effectuer certains tests, notamment de mesurer certains paramètres, et de prendre certains médicaments conformément à un programme établi par un médecin.

Toutes les valeurs des paramètres mesurés sont mises en mémoire et toutes les données mises en mémoire sont transmises à l'unité centrale.

Le document WO 80/02376 décrit un dispositif de traitement médical commandé par ordinateur, tel qu'un dispositif de traitement de sang par circulation extracorporelle. Ce dispositif comprend une pluralité d'organes pour la mise en circulation de liquides, en particulier, des pompes et des vannes, ainsi qu'une pluralité de capteurs pour mesurer des paramètres relatifs à la circulation des liquides (pression, débits) et pour tester le fonctionnement des organes de mise en circulation des liquides. Les signaux émis par les capteurs sont fournis à un ordinateur, qui, à partir de ces signaux et d'un programme de traitement, commande le fonctionnement des différents organes du dispositif.

Plusieurs dispositifs peuvent être reliés à un système de contrôle central au moyen duquel toutes les procédures se déroulant dans une même institution peuvent être contrôlées et, éventuellement, enregistrées. Le problème que pose le transfert des valeurs prises à chaque instant par une pluralité de paramètres n'est pas évoqué, ni même suggéré danc ce document.

Or, On a constaté que le contrôle continu de la machine de dialyse exige le transfert et la mise en mémoire d'une énorme quantité de valeurs pendant une durée relativement longue, de l'ordre de trois à quatre heures, correspondant à la durée totale du traitement. L'exploitation consécutive des valeurs obtenues entraîne une perte de temps considérable pour la machine comme pour le dispositif récepteur du fait du contrôle des nombreuses valeurs qui n'ont probablement pas changé durant toute la durée du traitement.

Ce que l'on vient d'exposer met en évidence la nécessité de pouvoir disposer d'un processus de gestion des paramètres relatifs à un traitement de dialyse qui permette successivement le transfert et l'exploitation de données numériques dans des temps beaucoup plus réduits qu'actuellement, afin de permettre l'utilisation dans un centre de contrôle d'une unité d'exploitation relativement simple et de puissance modeste et par conséquent relativement peu coûteuse.

Un objet de la présente invention est donc de proposer un procédé et une machine de dialyse qui permettent de satisfaire aux exigences ci-avant.

Pour atteindre cet objet, on prévoit, conformément à l'invention, un procédé de surveillance d'un traitement médical effectué au moyen d'une machine de dialyse reliée à une unité de contrôle central située en un lieu éloigné du lieu du traitement médical, le procédé comprenant les étapes de :
- mesurer et/ou élaborer, sur le lieu du traitement, une pluralité de paramètres indicatifs du fonctionnement de la machine de dialyse ;
- sélectionner, parmi la pluralité de paramètres, au moins un paramètres d'intérêt particulier ;
- appliquer, sur le lieu de traitement, au moins une règle de mémorisation aux valeurs prises par le paramètre sélectionné, pour sélectionner parmi ces valeurs un sous-groupe de valeurs à mettre en mémoire ;
- mettre en mémoire, sur le lieu du traitement, ce sous-groupe de valeurs ; et
- transférer le sous-groupe de valeurs à l'unité de contrôle central éloignée du lieu du traitement.

Selon une caractéristique de l'invention, la règle de mémorisation prescrit, à partir d'un domaine de valeurs admissibles préalablement déterminé pour le paramètre sélectionné, la mémorisation de la valeur du paramètre soit au moment où il sort du domaine de valeurs admissibles, soit au moment où il y rentre.

Selon une autre caractéristique de l'invention, la règle de mémorisation prescrit la mémorisation de l'instant auquel la valeur d'un paramètre sélectionné sort du domaine de valeurs admissibles ou y rentre.

Selon encore une autre caractéristique de l'invention, la règle de mémorisation prescrit la mémorisation des valeurs que prend le paramètre sélectionné à des intervalles de temps déterminés.

La présente invention a aussi pour objet une machine de dialyse comprenant :
- des moyens de mesure ou d'élaboration en continu d'une pluralité de paramètres indicatifs du fonctionnement de la machine de dialyse ;
- des premiers moyens de mémorisation pour mettre en mémoire des paramètres d'intérêt particulier sélectionnés parmi la pluralité de paramètres ;
- des seconds moyens de mémorisation pour mettre en mémoire au moins une règle de mémorisation des valeurs prises par chaque paramètre d'intérêt particulier ;
- des troisièmes moyens de mémorisation pour mettre en mémoire, conformément à la règle de mémorisation, des valeurs prises par les paramètres d'intérêt particulier ;
- des moyens de communication pour transférer, à une unité de contrôle central extérieure à la machine de dialyse, le contenu des troisièmes moyens de mémorisation ; et
- des moyens de d'exploitation pour recevoir les signaux émis par des moyens de mesure ou d'élaboration de paramètres, pour provoquer le stockage dans les moyens de mémorisation des informations correspondantes, et pour provoquer le transfert du contenu des troisièmes moyens de mémorisation, par les moyens de communication, à l'unité de contrôle central.

Selon une caractéristique de l'invention, la machine de dialyse comprend :
- des quatrièmes moyens de mémorisation pour mettre en mémoire des domaines de valeurs admissibles pour les paramètres d'intérêt ;
- des cinquièmes moyens de mémorisation pour mettre en mémoire au moins une valeur prises par un paramètre d'intérêt particulier, quand cette valeur est soit à l'intérieur soit à l'extérieur du domaine de valeurs admissibles.

Selon une autre caractéristique de l'invention, les moyens de d'exploitation sont prévus en outre pour provoquer le stockage, dans les cinquièmes moyens de mémorisation, de l'instant à laquelle une valeurs prise par un paramètre d'intérêt particulier sort du domaine de valeurs admissibles ou y ou rentre.

Selon encore une autre caractéristique de l'invention, les moyens de d'exploitation sont prévus en outre pour provoquer le transfert du contenu des cinquièmes moyens de mémorisation, par les moyens de communication, à l'unité de contrôle central.

Pour une meilleure compréhension de la présente invention, celle-ci sera décrite dans une forme préférée de réalisation, à simple titre d'exemple non limitatif et en se référant aux dessis ci-joints, selon lesquels :
- La figure 1 est un schéma de blocs fonctionnels représentant une machine de dialyse, dans lequel on a mis en évidence uniquement les éléments relatifs à la présente invention.
- Les figures 2 et 3 sont des schémas de séries d'instructions d'une forme préférée de réalisation du programme principal dans une unité arithmétique d'exploitation installée dans la machine de dialyse selon la figure 1.

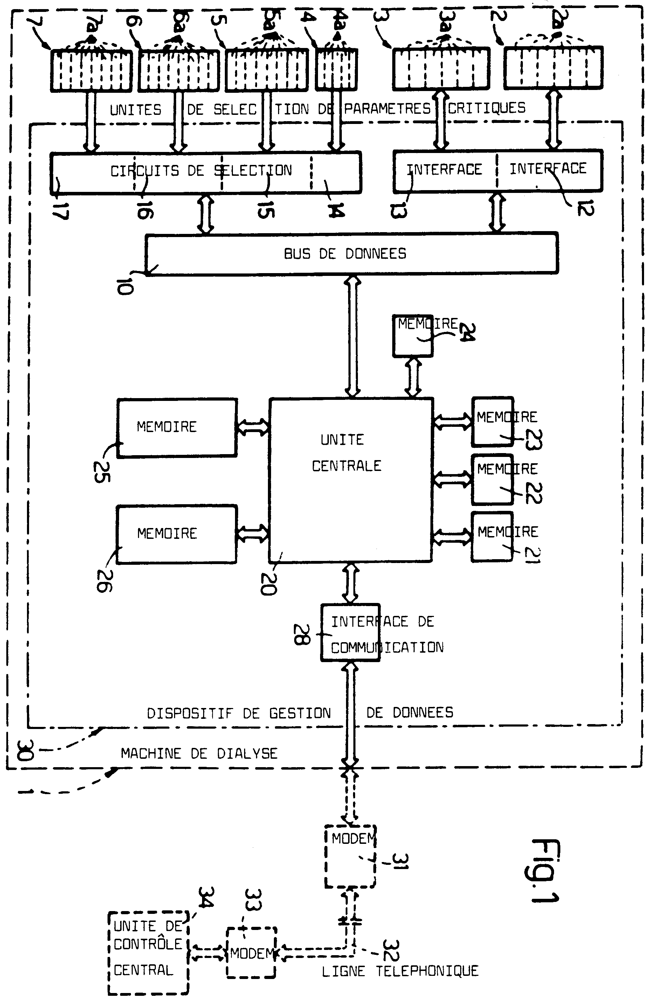

En se référant plus particulièrement à la figure 1, la référence 1 désigne l'ensemble d'une machine de dialyse dans laquelle les différents dispositifs qui exécutent ou contrôlent pratiquement la dialyse ne sont pas montrés dans la mesure où ils sont supposés déjà connus du technicien.

Au contraire, on met en évidence et l'on affecte des numéros 2 à 7 les unités de sélection des paramètres critiques de fonctionnement de la machine de dialyse 1.

Les unités 2 et 3 traitent respectivement les signaux de type analogique et digital et elles sont essentiellement du type actif dit aussi "intelligent" en ce qu'elles fournissent des valeurs déjà exploitées par elles-même ou par d'autres unités non représentées (comprenant par exemple des micro-processeurs disposés en différents endroits de la machine 1). Chaque unité 2, 3 peut traiter simultanément plusieurs paramètres, et présente ainsi plusieurs canaux de sélection, indiqués respectivement par les référence 2a et 3a.A titre d'exemple, les paramètres sélectionnés par les unités 2 et 3 pourraient concerner le liquide de dialyse (conductibilité, température, etc.).et/ou le dispositif d'ultrafiltration (diminution horaire de poids du patient, etc.).

Les unités 4,5,6,7 sont des unités de sélection passive de signaux de type respectivement digital (unités 4,5) et/ou de type analogique (unité 6) et du type comprenant des démodulateurs, à sortie numérique, des signaux utilisant un type quelconque de représentation (par exemple, modulation de fréquence, d'amplitude, de phase, ou bien modulation de la durée, de la position d'impulsions, ou bien encore, en général des transformations de spectres, des codes de protection ou de sécurité (unité 7).

L'unité 4 est constituée de préférence de microinterrupteurs de programmation (dip-switches) permettant une programmation manuelle de chaque fonction opérationnelle de la machine 1 de la part d'un spécialiste.

L'unité 5 est constituée essentiellement par une pluralité de capteurs du type "On/OFF" capables d'indiquer l"état d'un paramètre particulier (par exemple la pression du liquide de dialyse, une fuite de sang, etc) pendant un traitement de dialyse effectué par la machine 1.

L'unité 6 comprend de préférence des capteurs de type analogique capables d'émettre un signal dont l'amplitude dépend d'un paramètre correspondant sous contrôle (par exemple la pression veineuse, le débit d'une pompe à sang, etc.).

Les mêmes considérations valent pour l'unité 7, dont les capteurs sont capables d'engendrer un signal modulé ou codifié en fonction d'un paramètre correspondant.

Par analogie à ce que l'on vient de mentionner eu se référant aux unités 2,3, chacune des unités 4,5,6,7, traite d'autres paramètres et elles présentent des canaux de sélection indiqués respectivement par les références 4a, 5a, 6a, 7a.

Les unités 2 et 3 déja équipées d'une capacité d'élaboration locale, sont reliées à un "data bus 10" au moyen d'interfaces respectives 12, 13.

Les unités 4, 5, 6, 7 sont par contre reliées au "data bus" 10 au moyen de circuits de sélection respectifs et spécifiques 14, 15, 16, 17, essentiellement de types connus. Par exemple, les circuits 14, 15 pourraient comprendre une pluralité de circuits de type "tristate" aptes à un fonctionnement cyclique et connectés d'un côté aux capteurs de type digital cités ci-avant et d'un autre côté, à une ligne de transmission commune multipolaire reliée au "databus 10". Le circuit de sélection 16 pourrait comprendre un "multiplexer" de type analogique ayant des terminaux d'entrée reliés respectivement à des capteurs analogiques, et un convertisseur analogique/digital interposé entre le terminal de sortie du "multiplexer" et le "databus 10". Enfin, le circuit 17 présente des canaux dont chacun pourrait être constitué par un démodulateur numérique ayant une entrée apte à recevoir un signal modulé engendré par le capteur correspondant, et une sortie reliée au "data bus 10" par un convertisseur en mesure d'engendrer un signal de type logique (par exemple à 16 bits).

Le "data bus 10" est relié à une unité centrale d'exploitation 20, comprenant par exemple un microprocesseur qui échange des signaux avec une pluralité de dispositifs à mémoire 21, 22, 23, 24, 25, 26 selon les modalités exposées par la suite. L'unité 20 est également reliée à une interface de communication 28 à travers laquelle elle peut dialoguer avec l'extérieur. De préférence, l'interface 28 est équipée d'un dispositif de découplage galvanique (non représenté) dans le but de valider, dans le cas d'interconnexions avec d'autres appareils de communication (modem) ou d'exploitation prévues dans des conditions moins sévères, l'isolement et le rejet des dérangements pour lesquels la machine de dialyse a été étudiée.

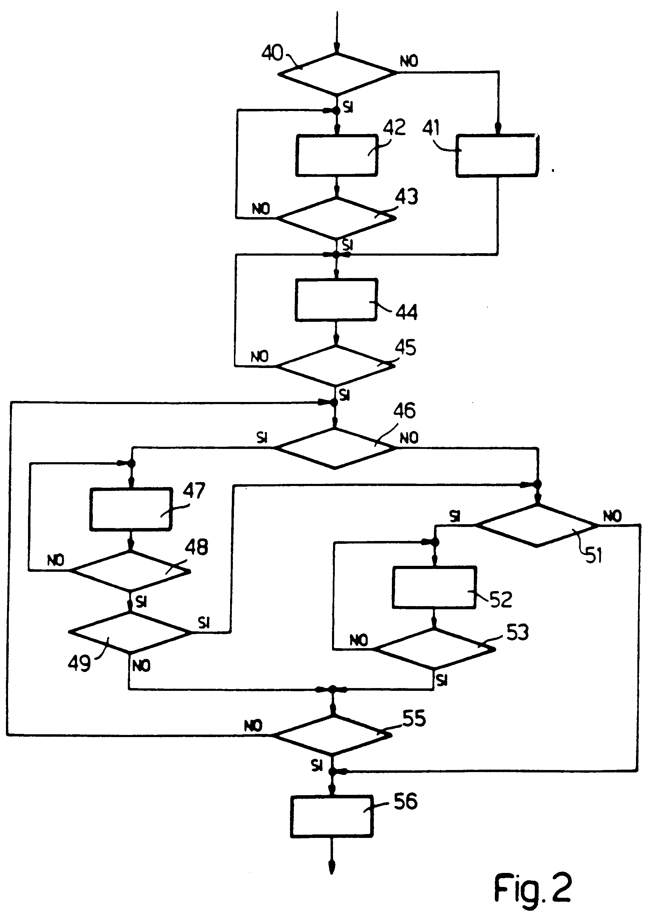

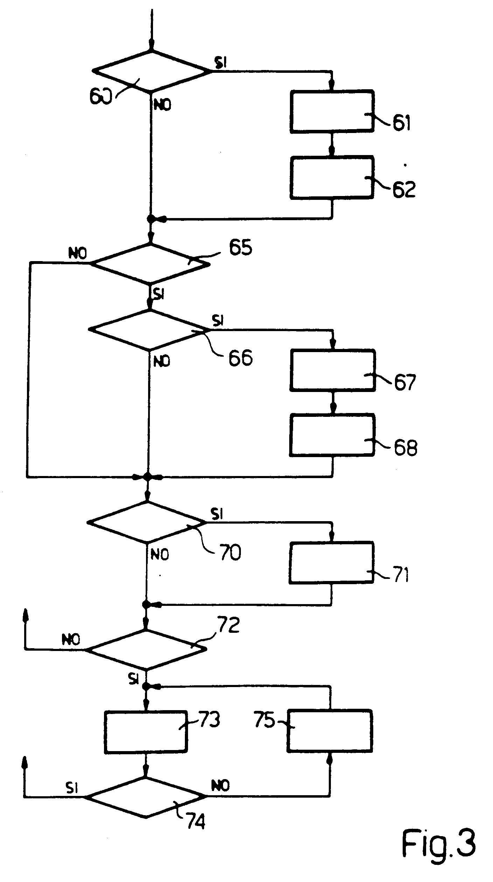

Les circuits 12 à 17, l'unité 20 avec les mémoires correspondantes 21 à 26 et l'interface série 28 constituent dans leur ensemble un dispositif 30 de gestion (sélection, élaboration, mise en mémoire, transfert) des signaux correspondants aux paramètres relatifs à un traitement de dialyse, dont les modalités de fonctionnement sont décrites par la suite en se référant aux figures 2 et 3. Cet ensemble est, en service normal, apte à dialoguer avec une unité de contrôle central 34, par exemple éloigné et utilisant des moyens appropriés d'interface télématique tels que des dispositifs modulateur - démodulateur 31,33 et une ligne téléphonique commune 32.

La figure 2 concerne le schéma d'une série d'instructions relative à quelques opérations préliminaires de mise au point que l'opérateur met en oeuvre our préparer le dispositif 30 à fonctionner lorsque commencera le traitement de dialyse.

On parvient d'abord à un bloc 40 où l'on vérifie si l'on a l'intention de régler l'horloge qui déterminera ensuite la chronologie de la sélection des valeurs par le dispositif 30. Dans le cas où l'on ne désire pas régler l'heure, on arrive au bloc 41 qui veille à fixer comme heure de départ de la dialyse par exemple : heures : 0 ; minutes : 0 ; secondes : 0. Dans le cas où l'on désire fixer une heure déterminée, par exemple celle à laquelle commence effectivement le traitement, on arrive à un bloc 42 prévu pour effectuer un tel affichage. On passe ensuite à un bloc 43 qui vérifie si la prédétermination de l'heure à été effectuée correctement. Dans la négative, on retourne à l'entrée du bloc 42, dans l'affirmative, on se rend, de même que depuis la sortie du bloc 41, au bloc 44.

Le bloc 44 a le devoir d'effectuer une sélection de canaux 2a, 3a, 4a, 5a, 6a, 7a de la figure 1 dans le but de retenir seulement ceux qui seront traversés par des signaux présentant de l'intérêt justifiant une mise en mémoire. Le bloc 44 sert aussi à mettre en mémoire les canaux sélectionnés à l'intérieur de la mémoire 21. Le bloc 45 vérifie l'exactitude de la sélection de ces canaux. En cas de sélection incorrecte, le bloc 45 exige une nouvelle intervention jusqu'à ce que la sélection soit correcte.

Lorsque la sélection est correcte, on arrive à un bloc de décision 46 où l'on vérifie l'intention de l'opérateur de faire mettre en mémoire, pendant le traitement de dialyse, des paramètres critiques de dialyse dans la mémoire 25, en particulier les conditions ou seuils limites d'au moins un des paramètres sélectionnés.

Dans l'affirmative, on arrive à un bloc 47 où il faut définir, pour chaque canal mentionné ci-dessus, un domaine de valeurs admissibles délimitant chaque domaine par des seuils limites et mettant en mémoire les seuils eux-mêmes à l'intérieur de la mémoire 22 de la figure 1. La vérification de la sélection correcte des seuils limites est effectuée au moyen d'un bloc 48, qui, en cas de sélection imcorrecte, exige une nouvelle intervention au niveau du bloc 47.

Lorsque la sélection est correcte, on arrive à un bloc de vérification 49 qui vérifie l'intention de l'opérateur de faire effectuer pendant le traitement de dialyse une mise en mémoire périodique des valeurs relevées pour les paramètres de la dialyse. Dans l'affirmative, on retourne à la sortie du bloc 46 et à l'entrée d'un bloc supplémentaire 51 de vérification. Celui-ci vérifie l'intention de l'opérateur de faire mettre en mémoire périodiquement pendant le traitement de dialyse, quelques uns des paramètres mémorisés dans la mémoire 26.

Dans l'affirmative, on arrive à un bloc 52 grâce auquel on définit, pour chacun des canaux concernés, les modalités de sélection et de mise en mémoire du paramètre correspondant. Par exemple on spécifie les valeurs relatives à la fréquence des mesures périodiques, au volume de mémoire à utiliser pour une mise en mémoire dans chaque canal,à d'éventuels coefficients pour effectuer, si nécessaire une transformation linéaire des signaux analogiques ou digitaux relevés, etc. Toutes ces valeurs citées ci-avant sont mémorisées dans la mémoire 23 de la figure 1. La vérification de l'enregistrement correct des valeurs citées ci-avant est effectuée à l'aide d'un bloc 53 qui, en cas d'enregistrement incorrect, exige une opération ultérieure faisant intervenir à nouveau le bloc 52.

En cas de disposition correcte, on arrive, de même que depuis la sortie du bloc 49, à l'entrée d'un bloc 55 qui vérifie si l'on a au moins effectué une opération de mise en mémoire des valeurs à l'intérieur des mémoires 21, 22, 23 de la figure 1. Dans la négative, ce qui signifie que, bien qu'ayant suivi une voie qui prévoyait une opération de sélection et de mise en mémoire des valeurs relevées pour les paramètres du traitement de dialyse, il n'y a pas eu d'opération effective (les mémoires 21, 22, 23 étant en fait vides), on retourne donc à l'entrée du bloc 46. Dans l'affirmative, on arrive, en même temps que de la sortie du bloc 51, à un bloc 56 qui commande le démarrage du traitement de dialyse par la machine 1.

La figure 3 concerne le schéma d'une série d'instructions qui est essentiellement suivie de manière cyclique par l'unité 20 pendant la durée du traitement de dialyse, dans le but de relever les faleurs des paramètres sélectionnés dans des conditions de fonctionnement particulière.

On arrive d'abord à un bloc de comparaison 60 qui vérifie si un ou plusieurs des paramètres sélectionnés ont une valeur en dehors du domaine des valeurs admissibles et mises en mémoire dans le dispositif 22.

Dans l'affirmative, on passe au bloc 61 qui met en mémoire dans la mémoire 25 au moins un des paramètres ayant franchi un seuil limite défini dans le bloc 47 de la figure 2 et en outre, également la date de l'évènement. A simple titre d'exemple, le dispositif mémoire 25 pourrait être de type circulaire, ou à échelons, afin de permettre la mise en mémoire de 64 groupes de paramètres, dans ce cas la mise en mémoire d'un 65e entraînerait automatiquement l'effacement de celui mis en mémoire le 1er par ordre chronologique.

Le paramètre (ou les paramètres) dont la valeur est sortie du domaine défini par les seuils limites, est ensuite mis en mémoire au moyen du bloc 62 à l'intérieur du dispositif à mémoire 24 de la figure n° 1, afin de pouvoir en contrôler le retour de la valeur à l'intérieur du domaine correspondant des valeurs admissibles.

Le contrôle du retour mentionné ci-avant est effectué à l'aide d'une série de blocs 65, 66, 67, 68. On parvient à l'entrée du premier d'entre eux directement depuis les sorties des blocs 60 et 62. En particulier, le bloc 65 vérifie s'il existe au moins un paramètre mis en mémoire à l'intérieur de la mémoire 24. Dans la négative, on passe à l'étape définie plus loin en relation au bloc 70, tandis que dans l'affirmative, on arrive à un bloc de comparaison 66 dont la fonction est de vérifier si la valeur du paramètre mesurée à cet instant est revenue ou non à l'intérieur du domaine des valeurs admissibles.

Dans la négative, on passe à l'étape définie plus loin en relation au bloc 70, tandis que dans l'affirmative, on effectue à l'aide du bloc 67, une mise en mémoire ultérieure des valeurs mesurées à cet instant d'au moins un des paramètres ayant franchi un seuil limite, ainsi que de l'heure de la mesure, d'une façon analogue à celle décrite ci-avant avec référence au bloc 61. On arrive ensuite au bloc 68 qui détermine l'abandon par la mémoire 24 du paramètre dont la valeur est rentrée à l'intérieur du domaine des valeurs admissibles.

Depuis les sorties des blocs 65, 66, 68, on arrive & un bloc de comparaison 70 qui vérifie si le moment est venu de mettre en mémoire des paramètres du traitement selon les modalités prévues au moyen du bloc 52 de la figure 2 et mise en mémoire dans la mémoire 23 de la figure 1. Dans la négative, on sort au-delà du bloc 70, tandis que dans l'affirmative, on arrive à un bloc 71 qui vérifie la mise en mémoire, à l'intérieur de la mémoire 26, des paramètres précités.

Des blocs 70 et 71, on arrive à l'entrée du bloc 72 qui recherche si, à travers l'interface de communication 28 de la figure 1 a été reçue une demande de transfert de valeurs mesurées, formulée par exemple par l'unité de contrôle centrale 34. Dans la négative, on poursuit les vérifications prévues dans le schéma de la figure 3, en recommençant par le bloc 60. Dans l'affirmative, on procède au transfert des valeurs vers l'unité 34 sous le contrôle du bloc 73, lequel par exemple, commande le transfert total des données contenues dans les mémoires 25 et/ou 26, ou bien le transfert de toutes les valeurs mesurées à un instant donné pour les paramètres de la dialyse qui ont été sélectionnés, selon la demande formulée par l'unité 34 elle-même.

Le contrôle de l'achèvement du transfert est effectué au moyen du bloc 74, lequel, dans l'affirmative, (transfert terminé de manière correcte) renvoie à l'entrée du bloc 60, pendant que, dans la négative (transfert en cours ou incorrect) il renvoie à l'entrée du bloc 73 au moyen d'un bloc d'attente 75 (analyse de la réponse).

Les opérations décrites ci-avant, en référence aux blocs 73,74,75 sont gérées par l'unité 28, afin de garantir une méthodologie sûre de transfert de données selon un protocole protégé, car il est extrêmement important que le transfert de données soit effectué de façon très fiable. Un ensemble de données pourrait être par exemple expédié à des unités extérieures intelligentes, qui & leur tour, pourraient s'entretenir avec d'autres unités intelligentes ou retransmettre un ensemble ultérieur de données au dispositif 30 avec des tâches de mise à disposition et/ou de mise en oeuvre et/ou d'interrogation.

De l'analyse du procédé de gestion et de la machine de dialyse selon la présente invention en découlent d'une manière évidente les avantages que l'on peut obtenir.

Avant tout, on observe que les informations relatives aux événements les plus significatifs (franchissement du seuil de domaine de valeurs admissibles) sont enregistrées et rendues immédiatement disponibles à l'intérieur d'une zone de mémoire bien spécifique.

D'autres informations concernant l'évolution des paramètres pendant le traitement de dialyse sont sélectionnés selon des modalités prédéterminées (par exemple périodiquement) et rendues également facilement disponibles et transférables depuis une autre zone de mémoire spécifique.

On n'exige donc pas l'emploi d'unités de contrôle éloignées dotées de capacités élevées de mémoire et de moyens d'exploitation. Au contraire, on observe qu'une unité de contrôle de capacité limitée, par exemple, un micro-ordinateur, peut ainsi gérer une pluralité de machines de dialyse (proches ou éloignées) qui effectuent des traitements simultanés et peut rendre disponibles dans une mémoire centrale (data base) les données reçues..

La possibilité de présélectionner les paramètres à contrôler et/ou à mettre en mémoire permettent d'envisager des stratégies de contrôle différentes d'un patient à l'autre et rend l'emploi de la machine de dialyse équipée selon la présente invention très souple et apte à faire face à des exigences opérationnelles les plus variées.

Il est clair enfin qu'au procédé et à la machine décrits ci-avant peuvent être apportées diverses modifications et variantes sans sortir de la portée de la présente invention.

## Revendications

1. Procédé de surveillance d'un traitement médical effectué au moyen d'une machine de dialyse reliée à une unité de contrôle central (34) située en un lieu éloigné du lieu du traitement médical, le procédé comprenant les étapes de :
- mesurer et/ou élaborer, sur le lieu du traitement, une pluralité de paramètres indicatifs du fonctionnement de la machine de dialyse ;
- sélectionner, parmi la pluralité de paramètres, au moins un paramètres d'intérêt particulier ;
- appliquer, sur le lieu de traitement, au moins une règle de mémorisation aux valeurs prises par le paramètre sélectionné, pour sélectionner parmi ces valeurs un sous-groupe de valeurs à mettre en mémoire ;
- mettre en mémoire, sur le lieu du traitement, ce sous-groupe de valeurs ; et
- transférer le sous-groupe de valeurs à l'unité de contrôle central (34) éloignée du lieu du traitement.

2. Procédé selon la revendication 1, caractérisé en ce que la règle de mémorisation prescrit, à partir d'un domaine de valeurs admissibles préalablement déterminé pour le paramètre sélectionné, la mémorisation de la valeur du paramètre au moment où il sort du domaine de valeurs admissibles.

3. Procédé selon la revendication 1, caractérisé en ce que la règle de mémorisation prescrit, à partir d'un domaine de valeurs admissibles préalable ment déterminé pour le paramètre sélectionné, la mémorisation de la valeur du paramètre au moment où il rentre dans le domaine de valeurs admissibles.

4. Procédé selon une des revendications 2 et 3, caractérisé en ce que la règle de mémorisation prescrit la mémorisation de l'instant auquel la valeur d'un paramètre sélectionné sort du domaine de valeurs admissibles ou y rentre.

5. Procédé selon une des revendications 2 à 4, caractérisé en ce que la règle de mémorisation prescrit en outre la mémorisation des valeurs que prend le paramètre sélectionné à des intervalles de temps déterminés.

6. Procédé selon une des revendications 2 à 5, caractérisé en ce que les paramètres sélectionnés sont choisis parmi les paramètres suivants : conductivité du liquide de dialyse, température du liquide de dialyse, pression du liquide de dialyse, quantité d'ultrafiltrat éliminée, présence de sang dans le circuit de liquide de dialyse, pression dans le circuit de restitution de sang, débit d'une pompe a sang.

7. Procédé selon une des revendications 2 à 5, caractérisé en ce que transfert du sous-groupe de valeurs à l'unité de contrôle central (34) est effectué par une ligne téléphonique.

8. Machine de dialyse comprenant :
- des moyens de mesure ou d'élaboration en continu (2, 3, 5, 6) d'une pluralité de paramètres indicatifs du fonctionnement de la machine de dialyse ;
- des premiers moyens de mémorisation (21) pour mettre en mémoire des paramètres d'intérêt particulier sélectionnés parmi la pluralité de paramètres ;
- des seconds moyens de mémorisation (23) pour mettre en mémoire au moins une règle de mémorisation des valeurs prises par chaque paramètre d'intérêt particulier ;
- des troisièmes moyens de mémorisation (26) pour mettre en mémoire, conformément à la règle de mémorisation, des valeurs prises par les paramètres d'intérêt particulier ;
- des moyens de communication (28) pour transférer, à une unité de contrôle central (34) extérieure à la machine de dialyse, le contenu des troisièmes moyens de mémorisation (26) ; et
- des moyens de d'exploitation (20) pour recevoir les signaux émis par des moyens de mesure ou d'élaboration (2, 3, 5, 6) de paramètres, pour provoquer le stockage dans les moyens de mémorisation (21, 23, 26) des informations correspondantes, et pour provoquer le transfert du contenu des troisièmes moyens de mémorisation (26), par les moyens de communication (28), à l'unité de contrôle central (34).

9. Machine de dialyse selon la revendication 8, caractérisée en ce qu'elle comprend :
- des quatrièmes moyens de mémorisation (22) pour mettre en mémoire des domaines de valeurs admissibles pour les paramètres d'intérêt ;
- des cinquièmes moyens de mémorisation (25) pour mettre en mémoire au moins une valeur prises par un paramètre d'intérêt particulier, quand cette valeur est soit à l'intérieur soit à l'extérieur du domaine de valeurs admissibles.

10. Machine de dialyse selon la revendication 9, caractérisée en ce que les moyens de d'exploitation (20) sont prévus en outre pour provoquer le stockage, dans les cinquièmes moyens de mémorisation (25), de l'instant à laquelle une valeur prise par un paramètre d'intérêt particulier sort du domaine de valeurs admissibles ou y rentre.

11. Machine de dialyse selon une des revendications 9 et 10, caractérisée en ce que les moyens de d'exploitation (20) sont prévus en outre pour provoquer le transfert du contenu des cinquièmes moyens de mémorisation (26), par les moyens de communication (28), à l'unité de contrôle central (34).

12. Machine de dialyse selon une des revendication 8 à 11, caractérisée en ce qu'une règle de mémorisation des valeurs prises par un paramètre d'intérêt particulier comprend la fréquence de la mesure ou de l'élaboration de la valeur ce paramètre.

13. Machine de dialyse selon une des revendication 8 à 12, caractérisée en ce que une règle de mémorisation des valeurs prises par un paramètre d'intérêt particulier comprend la définition de la place de mémoire allouée au stockage de ces valeurs.

14. Machine de dialyse selon une des revendication 8 à 13, caractérisée en ce que les paramètres mesurés comprennent la conductivité du liquide de dialyse, la température du liquide de dialyse, la pression du liquide de dialyse, la quantité d'ultrafiltrat éliminée, la présence de sang dans le circuit de liquide de dialyse, la pression dans le circuit de restitution de sang, le débit d'une pompe à sang.

## Claims

1. Method for monitoring a medical treatment carried out by means of a dialysis machine which is connected to a central control unit (31) located at a place remote from the place of the medical treatment, the method comprising the steps of:
- measuring and/or elaborating at the treatment place a plurality of parameters indicative of the operation of the machine;
- selecting among the plurality of parameters at least one parameter of special interest ;
- applying a rule of memorization, at the treatment place, to the values of the selected parameter to select from these values a subset of values to be stored ;
- storing, at the treatment place, this subset of values ; and
- transferring the subset of values to the central control unit (34) remote from the treatment place.

2. Method according to claim 1, characterized in that the rule of memorization prescribes, for a range of admissible values for the selected parameter, the memorization of the value of the parameter whenever it leaves the range of admissible values.

3. Method according to claim 1, characterized in that the rule of memorization prescribes, for a range of admissible values for the selected parameter, the memorization of the value of the parameter whenever it enters the range of admissible values.

4. Method according to one of the claims 2 and 3, characterized in that the rule of memorization prescribes the memorization of the time when the value of a selected parameter enters or leaves the range of admissible values.

5. Method according to one of the claims 2 to 4, characterized in that the rule of memorization further prescribes the memorization of the value of the selected parameter at determined time intervals.

6. Method according to one of the claims 2 to 5, characterized in that the selected parameters are chosen from the following parameters: conductivity of the dialysis liquid, temperature of the dialysis liquid, pressure of the dialysis liquid, amount of discarded ultrafiltrate, presence of blood in the dialysis liquid circuit, pressure in the blood return circuit, flowrate of a blood pump.

7. Method according to one of the claim 2 to 5, characterized in that the transfer of the subset of values to the central control unit (34) is carried out through a telephone line.

8. Dialysis machine comprising :
- means (2, 3, 5, 6) for continuously measuring or elaborating a plurality of parameters indicative of the operation of the dialysis machine;
- first memorization means (21) for storing parameters of special interest selected from the plurality of parameters;
- second memorization means (23) for storing at least a rule of memorization of values of each parameter of special interest;
- third memorization means (26) for storing, according to the memorization rule, values of the parameters of special interest;
- communication means (28) for transferring to a central control unit (34) outside the dialysis machine, the content of the third memorization means (26); and
- processing means (20) for receiving signals emitted by the means (2, 3, 5, 6) for continuously measuring or elaborating parameters, for causing the storage in the memorization means (21, 23, 26) of corresponding information, and for causing the transfer of the content of the third memorization means (26), by the communication means (28), to the central control unit (34).

9. Dialysis machine according to claim 8, characterized in that it further comprises:
- fourth memorization means (22) for storing ranges of admissible values for the parameters of interest;
- fifth memorization means (25) for storing at least a value of a parameter of special interest whenever this value is either inside or outside the range of admissible values.

10. Dialysis machine according to claim 9, characterized in that the processing means (20) is further apt to cause the storage, in the fifth memorization means (25) of the time when the value of a parameter of special interest enters or leaves the range of admissible values.

11. Dialysis machine according to one of the claims 9 and 10, characterized in that the processing means (20) is further apt to cause the transfer of the content of the fifth memorization means (26), by the communication means (28), to the central control unit (34).

12. Dialysis machine according to one of the claims 8 to 11, characterized in that a rule of memorization of the values of a parameter of special interest includes the frequency of the measurement or of the elaboration of the value of this parameter.

13. Dialysis machine according to one of the claims 8 to 12, characterized in that a rule of memorization of the values of a parameter of special interest includes the definition of the size of the memory allocated to the storage of these values.

14. Dialysis machine according to one of the claims 8 to 13, characterized in that the measured parameters comprise the conductivity of the dialysis liquid, the temperature of the dialysis liquid, the pressure of the dialysis liquid, the amount of discarded ultrafiltrate, the presence of blood in the dialysis liquid circuit, the pressure in the blood return circuit, the flow rate of a blood pump.

## Patentansprüche

1. Verfahren zur Überwachung einer medizinischen Behandlung, die mittels einer Dialysevorrichtung vorgenommen wird, welche mit einer zentralen Überwachungseinheit (34) verbunden ist, die sich an einem vom Ort der medizinischen Behandlung entfernten Ort befindet, wobei das Verfahren die folgenden Schritte aufweist :
- Messen und/oder Verarbeiten einer Mehrzahl das Betriebsverhalten der Dialysevorrichtung anzeigender Parameter am Ort der Behandlung;
- Auswählen wenigstens eines besonders interessierenden Parameters aus der Mehrzahl der Parameter;
- Anwenden wenigstens einer Speicherregel auf die von dem ausgewählten Parameter angenommenen Werte am Ort der Behandlung, um aus diesen Werten eine Untergruppe zu speichernder Werte auszuwählen;
- Speichern dieser Untergruppe von Werten am Ort der Behandlung; und
- Übertragen der Untergruppe von Werten zu der vom Ort der Behandlung entfernten zentralen Überwachungseinheit (34).

2. Verfahren nach Anspruch 1, dadurch gekennzeichnet, daß die Speicherregel aufgrund eines zuvor bestimmten Bereichs zulässiger Werte des ausgewählten Parameters die Speicherung des Parameterwertes vorschreibt, sobald er den Bereich zulässiger Werte verläßt.

3. Verfahren nach Anspruch 1, dadurch gekennzeichnet, daß die Speicherregel aufgrund eines zuvor bestimmten Bereichs zulässiger Werte des ausgewählten Parameters die Speicherung des Parameterwertes vorschreibt, sobald er in den Bereich zulässiger Werte zurückkehrt.

4. Verfahren nach einem der Ansprüche 2 und 3, dadurch gekennzeichnet, daß die Speicherregel die Speicherung des Zeitpunkts vorschreibt, zu dem der Wert eines ausgewählten Parameters den Bereich zulässiger Werte verläßt oder dorthin zurückkehrt.

5. Verfahren nach einem der Ansprüche 2 bis 4, dadurch gekennzeichnet, daß die Speicherregel darüberhinaus die Speicherung der Werte vorschreibt, die der ausgewählte Parameter in bestimmten Zeitabständen annimmt.

6. Verfahren nach einem der Ansprüche 2 bis 5, dadurch gekennzeichnet, daß die ausgewählten Parameter aus den folgenden Parametern ausgewählt werden: Leitfähigkeit der Dialyseflüssigkeit, Temperatur der Dialyseflüssigkeit, Druck der Dialyseflüssigkeit, Menge des entfernten Ultrafiltrats, Vorhandensein von Blut in dem Kreislauf der Dialyseflüssigkeit, Druck in dem Kreislauf der Blutrückgabe, Durchfluß einer Blutpumpe.

7. Verfahren nach einem der Ansprüche 2 bis 5, dadurch gekennzeichnet, daß die Übertragung der Untergruppe von Werten zu der zentralen Überwachungseinheit (34) über eine Telefonleitung durchgeführt wird.

8. Dialysevorrichtung mit:
- Mitteln zum kontinuierlichen Messen oder Verarbeiten (2, 3, 5, 6) einer Vielzahl den Betriebszustand der Dialysevorrichtung anzeigender Parameter;
- erste Speichermittel (21) zum Speichern von besonders interessierenden Parametern, die aus der Vielzahl von Parametern ausgewählt wurden;
- zweite Speichermittel (23) zum Speichern wenigstens einer Regel für das Abspeichern der von den besonders interessierenden Parameter angenommenen Werte;
- dritte Speichermittel (26) zum Speichern der von den besonders interessierenden Parametern angenommenen Werte nach Maßgabe der Speicherregel;
- Verbindungsmittel (28) zum Übertragen des Inhalts der dritten Speichermittel (26) zu einer zentralen Überwachungseinheit (34) außerhalb der Dialysevorrichtung;
- Auswertemittel (20) zum Empfangen der von den Parametermeß- oder -verarbeitungsmittel (2, 3, 5, 6) ausgesendeten Signale, zum Auslösen die Speicherung der zugehörigen Informationen in den Speichermitteln (21, 23, 26) und zum Auslösen die Übertragung des Inhalts des dritten Speichermittels (26) zu der zentralen Überwachungseinheit (34) durch die Verbindungsmittel (28).

9. Dialysevorrichtung nach Anspruch 8, dadurch gekennzeichnet, daß sie folgendes aufweist:
- vierte Speichermittel (22) zum Speichern von Bereichen zulässiger Werte für die interessierenden Parameter;
- fünfte Speichermittel (25) zum Speichern wenigstens eines von einem besonders interessierenden Parameter angenommenen Wertes, wenn dieser Wert entweder innerhalb oder außerhalb des Bereichs zulässiger Werte liegt.

10. Dialysevorrichtung nach Anspruch 9, dadurch gekennzeichnet, daß die Auswertemittel (20) außerdem dafür vorgesehen sind, in dem fünften Speichermittel (25) die Speicherung des Zeitpunkts auszulösen, zu dem ein von einem besonders interessierenden Parameter angenommener Wert den Bereich zulässiger Werte verläßt oder dorthin zurückkehrt.

11. Dialysevorrichtung nach einem der Ansprüche 9 und 10, dadurch gekennzeichnet, daß die Auswertemittel (20) außerdem dafür vorgesehen sind, die Übertragung des Inhalts des fünften Speichermittels (26) zu der zentralen Überwachungseinheit (34) durch die Verbindungsmittel (28) auszulösen.

12. Dialysevorrichtung nach einem der Ansprüche 8 bis 11, dadurch gekennzeichnet, daß eine Regel zur Speicherung der von einem besonders interessierenden Parameter angenommenen Werte die Häufigkeit der Messung oder der Verarbeitung des Wertes dieses Parameters umfaßt.

13. Dialysevorrichtung nach einem der Ansprüche 8 bis 12, dadurch gekennzeichnet, daß eine Regel zur Speicherung der von einem besonders interessierenden Parameter angenommenen Werte die Definition des der Speicherung dieser Werte zugewiesenen Speicherplatzes umfaßt.

14. Dialysevorrichtung nach einem der Ansprüche 8 bis 13, dadurch gekennzeichnet, daß die gemessenen Parameter die Leitfähigkeit der Dialyseflüssigkeit, die Temperatur der Dialyseflüssigkeit, den Druck der Dialyseflüssigkeit, die Menge des entfernten Ultrafiltrats, das Vorhandensein von Blut in dem Kreislauf der Dialyseflüssigkeit, den Druck in dem Kreislauf der Blutrückgabe, den Durchfluß einer Blutpumpe umfassen.
